# EUROPEAN PATENT SPECIFICATION

(11) **EP 0 892 270 B1**
(45) Date of publication and mention of the grant of the patent: **03.09.2003**
(21) Application number: 98110569.5
(22) Date of filing: 09.06.1998
(51) Int. Cl.: G01N 33/483, A61B 5/15

(54) **A body fluid analysis system and a method for flushing and single-point calibration**
System zur Analyse körperliches Flüssigkeit und Verfahren für Spülung und einpunktliche Kalibrierung
Système d'analyse d'un liquide corporel et procédé de rinçage et seul point d'étalonnage

(30) Priority: 17.07.1997 SE 9702738
(43) Date of publication of application: 20.01.1999
(73) Proprietor: Siemens-Elema AB, 171 95 Solna 1 (SE)
(72) Inventor: von Bahr, Pontus, 116 40 Stockholm (SE)

(56) References cited:
- US-A- 4 608 996
- US-A- 5 057 278
- US-A- 5 165 406

## Description

A body fluid analysis system and a method for flushing and single-point calibration

The present invention relates to a body fluid analysis system for extracorporeal body fluid analysis with a sensor means. The present invention also relates to a method for flushing and single-point calibration of a sensor means in a closed, body fluid analysis system.

In e.g. extracorporeal analysis of blood with known systems for continuous or semi-continuous blood analyses via a venous or arterial catheter, the heart or an external pump pumps the blood to a sensor outside the body. US-A-5 165 406 describes a body fluid analysis system, wherein after the blood analysis, performed by the sensor, the blood is returned to the body when a flushing liquid is pumped in the opposite direction. After a large part of the blood has been pumped back to the patient, the sensor is flushed with additional flushing liquid to remove any residual blood. This flushing liquid can either be infused into the patient or collected in a special bag. When flushing has been concluded, the flushing liquid, which is then in contact with the sensor, is sometimes used for calibration.

Instability and a short operating life are the main disadvantages of these known systems for blood analysis.

The plurality of pumps and plurality of bags/containers employed by these known systems are additional disadvantages.

The objective of the present invention is to solve the aforementioned problems. This is achieved with a body fluid analysis system for extracorporeal body fluid analysis using a sensor means according to claim 1.

The body fluid analysis system according to the present invention comprises a container, holding at least one flushing liquid, and a first tube connected to the container at a first point and a second point to form a closed system. The sensor means is connected to a second tube for connection to a patient. The body fluid analysis system also comprises a pump means connected to the first tube between the first point and the sensor means.

One advantage of the body fluid analysis system according to the present invention is that it can be realised at low cost. The system is also easy to operate. An additional advantage is that the system only needs one pump and one container. Moreover, the body fluid analysis system according to the present invention is stable and has a long operating life.

In conjunction herewith, it would be advantageous if the body fluid analysis system also comprised a valve means, connected to the first tube between the second point and the sensor means, the pump means and valve means being connected to the first tube on either side of the sensor means.

In conjunction herewith, it would be advantageous if the body fluid analysis system operated with counter-pressure in the container.

In conjunction herewith, an advantageous embodiment of the body fluid analysis system is achieved when the valve means is arranged by the sensor means and is also connected to the second tube.

An additional advantage is achieved in conjunction herewith when flow resistance in the two tubes is optimised by the choice of appropriate tube diameters and/or chokes using the valve means.

In conjunction herewith, it would be advantageous if a filter means for filtering out bacteria were connected to the first tube between the pump means and sensor means.

An additional advantage in conjunction herewith is achieved when the body fluid analysis system comprises analysis means, connected to the container, for determining contamination of the flushing liquid(s).

In conjunction herewith, an advantageous embodiment of the body fluid analysis system is achieved when the analysis means consists of a spectral analysis means or an absorption analysis means.

In conjunction herewith, it would be advantageous if the body fluid analysis system additionally contained an indication means, connected to the analysis means, for indicating when contamination of the flushing liquid(s) exceeds a redetermined level.

The method, according to the present invention, for flushing and single-point calibration of a sensor means in a closed, body fluid analysis system comprises the following steps:
- pumping body fluid to the sensor means;
- analysing the body fluid;
- pumping flushing liquid from a container through the sensor means in order to flush same; and
- collecting flushing liquid in the container after the sensor means is flushed.

In conjunction herewith, it would be advantageous if the method additionally comprised the following steps:
- closing a valve means at the same time as the pump means pumps towards the container in order to pump body fluid to the sensor means;
- keeping the valve means closed, and the pump means inactive while the sensor means analyses body fluids;
- keeping the valve means closed at the same time as the pump means pumps towards the sensor means in order to return body fluid to the body fluid source; and
- opening the valve means at the same time as the pump means pumps towards the sensor means in order to pump flushing liquid through the sensor means.

An additional advantage is achieved in conjunction herewith when the method additionally comprises the following step:
- a filter means filters out bacteria in the body fluid or flushing liquid respectively.

In conjunction herewith, it would be advantageous if the method additionally comprised the following step:
- after the flushing stage, flushing liquid is used for single-point calibration of the sensor means.

The invention will now be explained in greater detail, referring to attached drawings, in the following description of preferred embodiments of same.
FIG. 1 shows a body fluid analysis system according to the present invention;
FIG. 2 is a schematic view of an alternative embodiment of the valve means in the body fluid analysis system shown in FIG. 1; and
FIG. 3 is a flow chart depicting a method, according to the invention, for flushing and single-point calibration of a sensor means in a closed, body fluid analysis system.

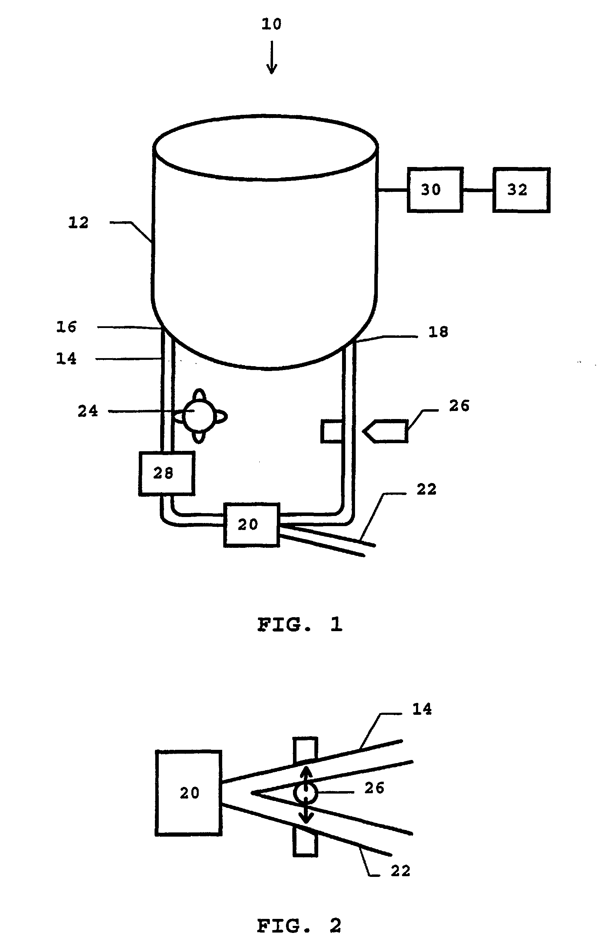

FIG. 1 shows a body fluid analysis system 10, according to the present invention, for extracorporeal body fluid analysis. This body fluid analysis system 10 is preferably used for blood analyses and will henceforth be referred to as a blood analysis system, even if other body fluids can also be analysed with this system. The blood analysis system 10 comprises a container 12 holding at least one flushing liquid. The blood analysis system also comprises a first tube 14, connected to the container 12 at two different points, i.e. a first point 16 and a second point 18. The first tube's 14 connection points 16, 18 are arranged so recirculation develops in the system. These points 16, 18 are therefore separated at a specific distance from each other to keep them from opening onto the same point in the container 12. The container 12 and the first tube 14 accordingly form a closed system.

The blood analysis system 10 also comprises a sensor means 20, connected to the first tube 14 and to a second tube 22. The second tube 22 connects the system 10 to a patient's blood circulation via a venous or arterial catheter (not shown). The blood analysis system 10 also comprises a pump means 24, connected to the first tube 14 between the container 12 and the sensor means 20, and a valve means 26, connected to the first tube 14 between the container and the sensor means 20. As FIG. 1 shows, the pump means 24 and the valve means 26 are connected to the first tube 14 on either side of the sensor means 20. The pump means 24 is a double-action pump, i.e. it is able to pump in both directions. The valve means 26 can be any kind of ON/OFF valve or the equivalent. The blood analysis system 10 also comprises a filter means 28, connected to the first tube 14 between the pump means 24 and the sensor means 20, for filtering out bacteria in blood or flushing liquid(s) respectively. The blood analysis system 10 shown in FIG. 1 operates with counter-pressure in the container 12. FIG. 1 schematically depicts an analysis means 30, connected to the container 12, for analysing the level of contamination in the flushing liquid(s). This analysis means 30 can consist of e.g. a spectral analysis means or an absorption analysis means. An indication means 32 is, in turn, connected to the analysis means 30 in order to indicate when contamination of the flushing liquid(s) exceeds a predetermined level. The indication can consist of e.g. an acoustic or optical signal or a combination thereof. When the indication means 32 indicates that contamination of the flushing liquid(s) exceeds the predetermined level, the entire container 12 is replaced with a new container, or the flushing liquid(s) in the container 12 is/are replaced.

As regards contamination, it should be noted that only traces of residual blood can be carried to the container 12, so the same flushing liquid can be used for a long time.

In one version of the body fluid analysis system 10 shown in FIG. 1, the valve means 26 is excluded, and the system's container 12 does not utilise counter-pressure. If the pump means 24 stops or the pump means 24 starts pumping in reverse (towards the point 16), arterial pressure will force blood up into the sensor means 20 for a predetermined period of time. The pump means 24 will then pump hard in the opposite direction (towards the point 18), the pressure then generated exceeding arterial pressure, whereupon blood and flushing liquid are carried both to the patient and through 18 to the container 12.

A version with an ON/OFF valve means on the catheter is also possible. When the valve means is open and the pump means operates, some of the flushing liquid flows through the catheter. The sensor means is flushed when the valve means is closed and the pump means operates. When the valve means is open and the pump means is inactive, the heart pumps blood through the sensor means. This naturally assumes that there is no positive pressure in the container.

The container 12 can either be flexible and empty of gas or hold just enough air to allow the volume of liquid to increase somewhat without any ill effects. A gas-tight container 12 is needed for blood gas analysis, of course, so the volume of gas (CO₂ in particular) remains constant.

FIG. 2 is a schematic view of an alternative embodiment of and location for the valve means in the blood analysis system 10 shown in FIG. 1. This figure only shows the sensor means 20, the first tube 14 and the second tube 22 in the blood analysis system 10, in addition to the valve means 26'. As FIG. 2 shows, the valve means 26' is arranged by the sensor means 20 and is connected to and acts on both the first tube 14 and the second tube 22. As FIG. 2 schematically depicts, the valve means 26' is able to close off either of the tubes 14, 22, leaving the unclosed tube open.

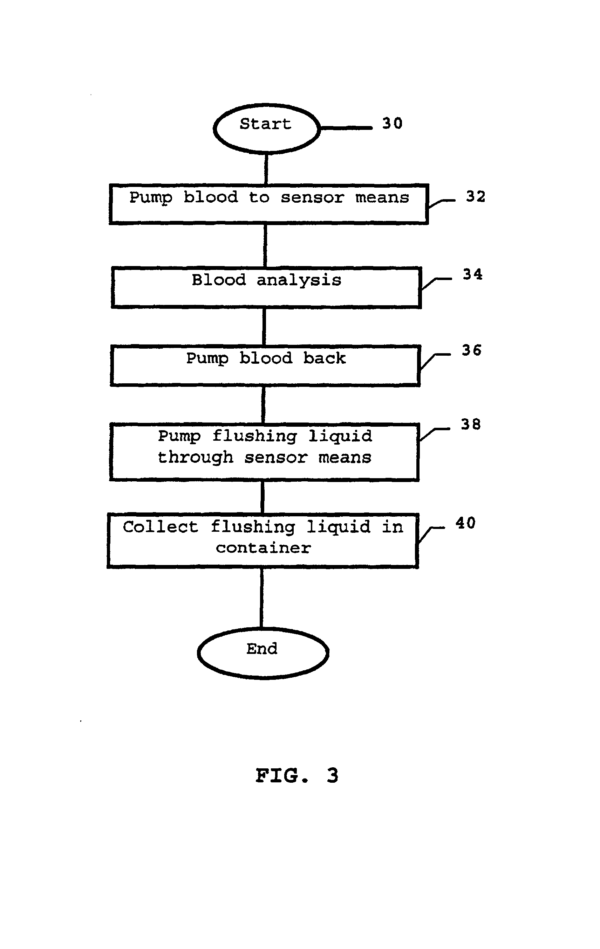

FIG. 3 is a flowchart showing a method, according to the invention, for flushing and single-point calibration of a sensor body in a closed, body fluid analysis system, e.g. devised according to FIG. 1. The method will be described below for blood, according to the description in FIG. 1, but any other body fluid can be substituted for blood. The method starts at block 30. A pump means at block 32 pumps blood to the sensor means. The sensor means performs a blood analysis at block 34. The pump means then pumps the blood at block 36 back to the blood source, i.e. the patient. At block 38, the pump means pumps flushing liquid from a container through the sensor means in order to flush same. After the sensor means has been flushed at block 40, flushing liquid is collected in the container. The method then concludes at block 42. Since the flow of flushing liquid is to and from the same container, the flushing liquid, which is initially sterile, becomes increasingly contaminated. But cleaning is still sufficient if the volume of the container is large enough. The method according to the invention also comprises the following steps:
- A valve means is closed at the same time the pump means pumps towards the container in order to pump blood to the sensor means. The valve means is then closed and the pump means is inactive while the sensor means performs the blood analysis. The valve means is then closed at the same time as the pump means pumps towards the sensor means in order to return blood to the blood source. The valve means is then open at the same time as the pump means pumps towards the sensor means in order to pump flushing liquid through the sensor means. In addition, a filter means filters bacteria out of the blood and flushing liquid respectively. After the flushing step, the flushing liquid is used for single-point calibration of the sensor means.

The above description of the method according to the present invention is also a description of the function of the blood analysis system shown in FIG. 1.

The invention is not limited to the depicted embodiments. A plurality of variations are possible within the framework of the attached claims.

## Claims

1. A body fluid analysis system (10) for extracorporeal body fluid analysis with a sensor means (20), **characterised in that** the system comprises a container (12), holding at least one flushing liquid, and a first tube (14), connected to the container (12) at a first point (16) and a second point (18) to form a closed system, the sensor means (20) is connected to the first tube (14), and to a second tube (22), for connection to a patient, and the body fluid analysis system (10) additionally comprises a pump means (24) connected to the first tube (14) between the first point (16) and the sensor means (20).

2. The body fluid analysis system according to claim 1, **characterised in that** the body fluid analysis system (10) additionally comprises a valve means (26), connected to the first tube (14) between the second point (18) and the sensor means (20), the pump means (24) and the valve means (26) being connected to the first tube (14) on either side of the sensor means (20).

3. The body fluid analysis system (10) according to claim 2, **characterised in that** a counter-pressure is present in the container (12).

4. The body fluid analysis system (10) according to any of claims 1-3, **characterised in that** the valve means (26') is arranged by the sensor means (20) and is also connected to the second tube (22).

5. The body fluid analysis system (10) according to any of claims 1-4, **characterised in that** flow resistance in both tubes (14, 22) are optimised by the appropriate choice of tube diameter and/or chokes with the valve means (26, 26').

6. The body fluid analysis system (10) according to any of claims 1-5, **characterised in that** a filter means (28) is connected to the first tube (14) between the pump means (24) and the sensor means (20) to filter out bacteria.

7. The body fluid analysis system (10) according to any of claims 1-6, **characterised in that** the system additionally comprises an analysis means (30), connected to the container (12), for analysing contamination of the flushing liquid(s).

8. The body fluid analysis system (10) according to claim 7, **characterised in that** the analysis means (30) consists of a spectral analysis means (30) or an absorption analysis means (30).

9. The body fluid analysis system (10) according to any of claims 7-8, **characterised in that** the system additionally comprises an indication means (32), connected to the analysis means (30), for indicating when contamination of the flushing liquid(s) exceeds a predetermined level.

10. The method for flushing and single-point calibration of a sensor means (20) in a closed, body fluid analysis system (10) according to any of claims 1-9, said method being **characterised by** the following steps:
- Body fluid is pumped to the sensor means (20);
- The sensor means (20) analyses the body fluid;
- A pump means (24) pumps the body fluid back to the body fluid source;
- The pump means (24) pumps flushing liquid from a container (12) through the sensor means (20) in order to flush same; and
- The flushing liquid is collected in the container (12) after the sensor means (20) has been flushed.

11. A method for flushing and single-point calibration according to claim 10, **characterised in that** the method additionally comprises the following steps:
- The pump means (24) pumps body fluid to the sensor means (20);
- A valve means (26; 26') is closed at the same time as the pump means (24) pumps towards the container (12) in order to pump body fluid to the sensor means (20);
- The valve means (26; 26') is closed, and the pump means (24) is inactive while the sensor means (20) performs a body fluid analysis;
- The valve means (26; 26') is closed at the same time as the pump means (24) pumps towards the sensor means (20) in order to pump body fluid back to the body fluid source; and
- The valve means 26; 26') is open at the same time as the pump means (24) pumps towards the sensor means (20) in order to pump flushing liquid through the sensor means (20).

12. The method for flushing and single-point calibration according to claim 10 or 11, said method additionally **characterised by** the following step:
- A filter means (28) filters bacteria out of the body fluid and flushing liquid respectively.

13. The method for flushing and single-point calibration according to any of claims 10-12, **characterised in that** the method additionally comprises the following step:
- Flushing liquid is used after the flushing step for single-point calibration of the sensor means (29).

## Patentansprüche

1. Ein Körperflüssigkeitsanalysensystem (10) für die extrakorporale Körperflüssigkeitsanalyse mit einem Sensormittel (20), **dadurch gekennzeichnet, dass** das System einen Behälter (12), der zumindest eine Spülflüssigkeit enthält, und einen ersten Schlauch (14), der mit dem Behälter (12) in einem ersten Punkt (16) und einem zweiten Punkt (18) verbunden ist, um ein geschlossenes System zu bilden, ausweist, wobei das Sensormittel (20) mit dem ersten Schlauch (14) und mit einem zweiten Schlauch (22) zur Verbindung mit einem Patienten verbunden ist und das Körperflüssigkeitsanalysensystem (10) zusätzlich ein Pumpenmittel (24) aufweist, das mit dem ersten Schlauch (14) zwischen dem ersten Punkt (16) und dem Sensormittel (20) verbunden ist.

2. Das Körperflüssigkeitsanalysensystem gemäß Anspruch 1, **dadurch gekennzeichnet, dass** das Körperflüssigkeitsanalysesystem (10) zusätzlich eine Ventilmittel (26) aufweist, das mit dem ersten Schlauch (14) zwischen dem zweiten Punkt (18) und dem Sensormittel (20) verbunden ist, wobei das Pumpenmittel (24) und das Ventilmittel (26) mit dem ersten Schlauch (14) beiderseits des Sensormittels (20) verbunden sind.

3. Das Körperflüssigkeitsanalysensystem (10) gemäß Anspruch 2, **dadurch gekennzeichnet, dass** ein Gegendruck in dem Behälter (12) vorhanden ist.

4. Ein Körperflüssigkeitsanalysensystem (10) gemäß einem der Ansprüche 1-3, **dadurch gekennzeichnet, dass** das Ventilmittel (26') bei dem Sensormittel (20) angeordnet und auch mit dem zweiten Schlauch (22) verbunden ist.

5. Das Körperflüssigkeitsanalysensystem (10) gemäß einem der Ansprüche 1-4, **dadurch gekennzeichnet, dass** der Flusswiderstand in beiden Schläuchen (14, 22) durch passende Wahl des Schlauchdurchmessers und/oder Drosseln mit dem Ventilmittel (26, 26') optimiert wird.

6. Das Körperflüssigkeitsanalysensystem (10) gemäß einem der Ansprüche 1-5, **dadurch gekennzeichnet, dass** ein Filtermittel (28) mit dem ersten Schlauch (14) zwischen dem Pumpenmittel (24) und dem Sensormittel (20) verbunden ist, um Bakterien herauszufiltern.

7. Das Körperflüssigkeitsanalysensystem (10) gemäß einem der Ansprüche 1-6, **dadurch gekennzeichnet, dass** das System zusätzlich ein Analysenmittel (30) aufweist, das mit dem Behälter (12) zum Analysieren von Kontamination der Spülflüssigkeit verbunden ist.

8. Das Körperflüssigkeitsanalysensystem (10) gemäß Anspruch 7, **dadurch gekennzeichnet, dass** das Analysenmittel (30) aus einem Spektralanalysenmittel (30) oder einem Absorptionsanalysenmittel (30) besteht.

9. Das Körperflüssigkeitsanalysensystem (10) gemäß einem der Ansprüche 7-8, **dadurch gekennzeichnet, dass** das System zusätzlich ein mit dem Analysenmittel (30) verbundenes Anzeigemittel (32) aufweist, um anzuzeigen, wenn die Kontamination der Spülflüssigkeit(en) ein vorbestimmtes Niveau übersteigt.

10. Das Verfahren zum Spülen und Einpunktskalibrieren eines Sensormittels (20) in einem geschlossenen Körperflüssigkeitsanalysensystem (10) gemäß einem der Ansprüche 1-9, wobei dieses Verfahren durch die folgenden Schritte **gekennzeichnet** ist:
- Körperflüssigkeit wird zu dem Sensormittel (20) gepumpt;
- das Sensormittel (20) analysiert die Körperflüssigkeit;
- ein Pumpenmittel (24) pumpt die Körperflüssigkeit zurück zu der Körperflüssigkeitsquelle;
- das Pumpenmittel (24) pumpt Spülflüssigkeit von einem Behälter (12) durch das Sensormittel (20), um dieses zu spülen; und
- die Spülflüssigkeit wird in dem Behälter (12) gesammelt, nachdem das Sensormittel (20) gespült worden ist.

11. Ein Verfahren zum Spülen und Einpunktskalibrieren gemäß Anspruch 10, **dadurch gekennzeichnet, dass** das Verfahren zusätzlich die folgenden Schritte aufweist:
- das Pumpenmittel (24) pumpt Körperflüssigkeit zu dem Sensormittel (20);
- ein Ventilmittel (26, 26') wird gleichzeitig damit geschlossen, dass das Pumpenmittel (24) in Richtung auf den Behälter (12) pumpt, um Körperflüssigkeit zu dem Sensormittel (20) zu pumpen;
- das Ventilmittel (26, 26') ist geschlossen und das Pumpenmittel (24) inaktiv, während das Sensormittel (20) eine Körperflüssigkeitsanalyse durchführt;
- das Ventilmittel (26, 26') wird gleichzeitig damit geschlossen, dass das Pumpenmittel (24) in Richtung auf das Sensormittel (20) pumpt, um Körperflüssigkeit zu der Körperflüssigkeitsquelle zurückzupumpen; und
- das Ventilmittel (26, 26') ist gleichzeitig damit offen, dass das Pumpenmittel (24) in Richtung auf das Sensormittel (20) pumpt, um Spülflüssigkeit durch das Sensormittel (20) zu pumpen.

12. Das Verfahren zum Spülen und Einpunktskalibrieren gemäß Anspruch 10 oder 11, wobei das Verfahren zusätzlich durch die folgenden Schritte **gekennzeichnet** ist:
- ein Filtermittel filtert Bakterien aus der Körperflüssigkeit beziehungsweise aus der Spülflüssigkeit heraus.

13. Das Verfahren zum Spülen und Einpunktskalibrieren gemäß einem der Ansprüche 10-12, **dadurch gekennzeichnet, dass** das Verfahren zusätzlich die folgenden Schritte aufweist:
- Spülflüssigkeit wird nach dem Spülschritt zur Einpunktkalibrierung des Sensormittels (29) verwendet.

## Revendications

1. Système (10) d'analyse d'un fluide corporel pour une analyse extracorporelle d'un fluide corporel, comprenant des moyens (20) formant capteur, **caractérisé en ce que** le système comprend une cuve (12) contenant au moins un liquide de rinçage et un premier tube (14) communiquant avec la cuve (12) en un premier point (16) et un deuxième point (18) pour former un système fermé, les moyens (20) formant capteur étant reliés au premier tube (14) et au deuxième tube (22) en vue d'une liaison avec un patient, et le système (10) d'analyse d'un fluide corporel comprenant en outre un moyen (24) de pompage communiquant avec le premier tube (14) entre le premier point (16) et les moyens (20) formant capteur.

2. Système d'analyse d'un fluide corporel suivant la revendication 1, **caractérisé en ce que** le système (10) d'analyse d'un fluide corporel comprend en outre une vanne (26) reliée au premier tube (14) entre le second point (18) et les moyens (20) formant capteur, le moyen (24) de pompage et la vanne (26) étant reliés au premier tube (14) de part et d'autre des moyens (20) formant capteur.

3. Système (10) d'analyse d'un fluide corporel suivant la revendication 2, **caractérisé en ce qu'**une contre-pression est présente dans la cuve (12).

4. Système (10) d'analyse d'un fluide corporel suivant l'une quelconque des revendications 1 à 3, **caractérisé en ce que** la vanne (26') se trouve sur les moyens (20) formant capteur et est reliée également au deuxième tube (22).

5. Système (10) d'analyse d'un fluide corporel suivant l'une quelconque des revendications 1 à 4, **caractérisé en ce que** la résistance hydraulique des deux tubes (14, 22) est optimisée par le choix approprié du diamètre des tubes et/ou des étranglements de la vanne (26, 26').

6. Système (10) d'analyse d'un fluide corporel suivant l'une quelconque des revendications 1 à 5, **caractérisé en ce qu'**un moyen (28) de filtration est relié au premier tube (14) entre le moyen (24) de pompage et les moyens (20) formant capteur pour séparer les bactéries par filtration.

7. Système (10) d'analyse d'un fluide corporel suivant l'une quelconque des revendications 1 à 6, **caractérisé en ce que** le système comprend en outre un moyen (30) d'analyse relié à la cuve (12) pour analyser la pollution du ou des liquides de rinçage.

8. Système (10) d'analyse d'un fluide corporel suivant la revendication 7, **caractérisé en ce que** le moyen (30) d'analyse consiste en un moyen (30) d'analyse de spectre ou en un moyen (30) d'analyse par absorption.

9. Système (10) d'analyse d'un fluide corporel suivant l'une quelconque des revendications 7 ou 8, **caractérisé en ce que** le système comprend en outre un moyen (32) indicateur relié au moyen (30) d'analyse pour indiquer lorsque la pollution du ou des liquides de rinçage dépasse un niveau déterminé à l'avance.

10. Procédé de rinçage et d'étalonnage à un seul point de moyens (20) formant capteur dans un système (10) fermé d'analyse d'un fluide corporel suivant l'une quelconque des revendications 1 à 9, le procédé étant **caractérisé par** les stades suivants:
- on envoie par pompage du fluide corporel aux moyens (20) formant capteur ;
- les moyens (20) formant capteur analysent le fluide corporel ;
- un moyen (24) de pompage retourne le fluide corporel par pompage à la source de fluide corporel ;
- le moyen (24) de pompage pompe du liquide de rinçage d'une cuve (12) dans les moyens (20) formant capteur pour les rincer ; et
- on recueillie le liquide de rinçage dans la cuve (12) après que les moyens (20) formant capteur ont été rincés.

11. Procédé de rinçage et d'étalonnage à un seul point suivant la revendication 10, **caractérisé en ce que** le procédé comprend en outre les stades suivants :
- le moyen (24) de pompage envoie du fluide corporel par pompage aux moyens (20) formant capteurs ;
- on ferme une vanne (26 ; 26') dans le même temps que le moyen (24) de pompage refoule vers la cuve (12) afin d'envoyer du fluide corporel par pompage aux moyens (20) formant capteur ;
- on ferme la vanne (26 ; 26') et on rend inactif le moyen (24) de pompage, tandis que les moyens (20) de capteur effectuent une analyse du fluide corporel ;
- on ferme la vanne (26 ; 26') dans le même temps que le moyen (24) de pompage refoule vers les moyens (20) formant capteur afin de renvoyer par pompage du fluide corporel à la source de fluide corporel ;
- on ouvre la vanne (26 ; 26') dans le même temps que le moyen (24) de pompage refoule vers les moyens (20) formant capteur afin d'envoyer par pompage du liquide de rinçage dans les moyens (20) formant capteur.

12. Procédé de rinçage et d'étalonnage à un seul point suivant la revendication 10 ou 11, le procédé étant **caractérisé en outre par** le stade suivant
- un moyen (28) de filtration élimine par filtration des bactéries du fluide corporel et du liquide de rinçage respectivement.

13. Procédé de rinçage et d'étalonnage à un seul point suivant l'une quelconque des revendications 10 à 12, **caractérisé en ce que** le procédé comprend en outre le stade suivant:
- on utilise du liquide de rinçage après le stade de rinçage pour l'étalonnage à un seul point des moyens (29) formant capteur.
